# FASCICULE DE BREVET EUROPEEN

(11) **EP 1 317 239 B1**
(45) Date de publication et mention de la délivrance du brevet: **12.04.2006**
(21) Numéro de dépôt: 00960786.2
(22) Date de dépôt: 04.09.2000
(51) Int. Cl.: A61K 8/97, A61Q 7/00

(54) **ASSOCIATIONS SYNERGIQUES A BASE DE PLANTES POUR TRAITER LA CHUTE DES CHEVEUX**
SYNERGISTISCHE PFLANZENMISCHUNG GEGEN HAARAUSFALL
SYNERGETIC COMBINATIONS BASED ON PLANTS FOR TREATING HAIR LOSS

(43) Date de publication de la demande: 11.06.2003
(73) Titulaire: Shrivastava, Ravi, 63118 Cebazat (FR)
(72) Inventeur: Shrivastava, Ravi, 63118 Cebazat (FR)
(74) Mandataire: Larcher, Dominique
(86) Numéro de dépôt international: PCT/FR2000/002432
(87) Numéro de publication internationale: WO 2002/019974

(56) Documents cités:
- WO-A-97/05887
- WO-A-98/33472
- FR-A- 2 791 255
- US-A- 5 407 675
- US-A- 5 972 345
- US-A- 5 981 543

## Description

La présente invention concerne l'utilisation d'une association d'extraits de plantes synergiques avec ou sans un promoteur d'absorption en application locale sur le cuir chevelu pour diminuer la chute des cheveux sans induire de toxicité locale ou générale et traiter l'alopécie androgénétique sans provoquer d'effets secondaires.

La perte des cheveux chez l'homme qui se manifeste par la calvitie, ou alopécie androgénétique, n'est pas une maladie mais un phénomène physiologique qui a une influence importante sur le physique et le comportement social. Comme de plus en plus de personnes souhaitent conserver une apparence jeune, la calvitie constitue pour eux un problème important dans notre civilisation moderne. Une étude épidémiologique mondiale récente a montré que 42% des hommes entre 18 et 49 ans souffrent d'une perte de cheveux modérée à importante.

Chaque cheveu est issu d'un follicule capillaire. Le follicule capillaire suit des transformations cycliques qui commencent par une phase de croissance (anagène) suivie d'une phase de repos (catagène) et se terminent par une phase d'élimination (télogène). Un cuir chevelu sain comporte 80 à 90% de cheveux en phase anagène, 1% en phase catagène et 10 à 20% en phase télogène. Une augmentation du pourcentage de cheveux en phase télogène correspond à une perte de cheveux anormale.

Le follicule capillaire est le tissu de notre corps qui a la croissance cellulaire la plus intense. Il présente un métabolisme utra-rapide, ce qui le rend très sensible. Le moindre déséquilibre peut ralentir ou arrêter complètement la pousse du cheveu.

Les études scientifiques récentes montrent que les hormones androgènes influencent la chute des cheveux. Au niveau du cuir chevelu, une enzyme particulière, la 5 alpha réductase, convertit la testostérone en dihydrotestostérone. La testostérone favorise la croissance des cheveux tandis que la dihydrotestostérone accélère la chute en raccourcissant la phase anagène. Il a ainsi été montré que la perte des cheveux est liée à la transformation excessive de la testostérone en dihydrotestostérone au niveau des follicules capillaires par la 5 alpha réductase.

Un régime déséquilibré, appauvri en vitamines et en minéraux peut également influencer la chute des cheveux. Les acides aminés, et notamment les acides aminés soufrés (cystine, cystéine, méthionine) sont indispensables à la pousse des cheveux. Les oligo-éléments comme le zinc, le cuivre, le fer, le magnésium et quelques rares vitamines (B5, B8) jouent également un rôle fondamental dans le processus de pousse des cheveux.

De nombreuses compositions diététiques ou pharmaceutiques utilisent des produits naturels ou synthétiques par voie orale afin de lutter contre la chute des cheveux mais leur efficacité reste très limitée.

Il existe ainsi des compositions diététiques ou des compléments alimentaires pour "nourrir les cheveux par la racine" mais ces produits n'ont pas d'effet sur les androgènes.

Quelques médicaments qui prétendent réduire la chute des cheveux selon divers degrés d'efficacité sont présents sur le marché. Le Minoxidil® (Laboratoires CS, France) et Propecia® (Finastéride®, Merck Sharp & Dohme, USA) sont considérés comme des références dans ce domaine.

Le Minoxidil® est une solution pour application locale (deux fois par jour pendant 6 à 12 mois) qui est indiquée pour le traitement de l'alopécie androgénétique. Le mode d'action du Minoxidil® est lié à une vasodilatation qui provoque une augmentation de la microcirculation locale et améliore l'apport de nutriments au niveau du bulbe capillaire. Ce type de produit n'inhibe pas la conversion de la testostérone en dihydrotestostérone et n'apporte pas aux cheveux les éléments indispensables à leur croissance.

Propecia® est un produit à base de Finastéride®, une molécule de synthèse. Ce médicament par voie orale est employé à forte dose pour obtenir des concentrations suffisantes au niveau des follicules capillaires. Les fortes concentrations sanguines nécessaires pour obtenir un effet sur les follicules capillaires sont très toxiques et provoquent de nombreux effets secondaires. Ce produit doit être utilisé sous contrôle médical.

Certains extraits de plantes inhibiteurs de la 5 alpha réductase ont également été utilisés en application locale, seuls ou éventuellement associés à des acides aminés, des vitamines et des minéraux. Comme la couche cornée de la peau forme une véritable barrière imperméable, les extraits de plantes et les nutriments ainsi apportés par voie locale sont très mal absorbés. Ainsi, ces agents n'atteignent pas les follicules capillaires et ces produits ont une très faible efficacité.

Il n'existe donc sur le marché aucun produit pour application locale destiné à inhiber spécifiquement la conversion de la testostérone en dihydrotestostérone qui traverse suffisamment la barrière cutanée au niveau du cuir chevelu pour être efficace et qui soit non toxique.

Or, pour lutter contre la chute des cheveux, il est indispensable de transporter les inhibiteurs de la conversion de la testostérone en dihydrotestostérone en concentration physiologiquement active au niveau même de leur site d'action sans perturber l'équilibre hormonal général.

Ainsi, un traitement idéal pour diminuer la chute des cheveux devrait posséder les qualités suivantes :
- Inhiber énergiquement la conversion de la testostérone en dihydrotestostérone au niveau du follicule capillaire en utilisant des transporteurs capables de faire traverser la barrière cutanée aux substances actives.
- Ne pas modifier l'équilibre hormonal général.
- Etre non toxique, ne pas provoquer d'irritation ou d'effets secondaires.

Or la demanderesse a découvert avec étonnement que la chute des cheveux peut être diminuée en un temps très court par l'utilisation d'une association d'extraits de plantes synergiques qui inhibent fortement la 5 alpha réductase en combinaison avec un promoteur d'absorption qui assure leur transport précisément au niveau des follicules capillaires, et ceci sans provoquer d'irritation, de toxicité locale ou générale.

L'association de différents extraits de plantes agissant spécifiquement et de façon synergique sur la conversion de la testostérone en dihydrotestostérone n'a jamais été décrite.

Cette stratégie innovante permet d'inhiber fortement l'enzyme responsable de la conversion de la testostérone, uniquement au niveau du cuir chevelu, d'agir sans perturber l'équilibre hormonal général et sans produire d'effets secondaires.

Une augmentation localisée des taux de testostérone stimule ainsi l'activité de synthèse, la croissance des cheveux et prolonge la durée de vie des follicules capillaires.

L'efficacité de cette association d'extraits de plantes par voie locale peut être encore améliorée par l'apport par voie orale des nutriments indispensables à la croissance du cheveu.

Certains médicaments utilisés pour traiter l'hypertrophie bénigne de la prostate comme Proscar® (Finastéride®, Merck Sharp & Dohme, USA) ou Permission® (fraction lipido stérolique d'un extrait de Pygeum africanum, Laboratoires Pierre Fabre, France) agissent principalement en inhibant la 5 alpha réductase de la prostate. Nous avons testé de très nombreux extraits de plantes traditionnellement utilisés pour traiter l'hypertrophie bénigne de la prostate et évalué leur activité inhibitrice vis à vis de la 5 alpha réductase à l'aide de techniques biochimiques et *in vitro*.

Il a été observé que certains extraits de plantes comme Serenoa repens, Urtica dioica, Cucurbita pepo ou Epilobium angustifolium inhibent fortement la 5 alpha réductase (entre 30 et 49%) et qu'il existe une synergie lorsque certains de ces extraits sont utilisés en association. Certaines associations de trois extraits de plantes comme Serenoa repens (1/3) avec Urtica (1/3) dioica et Cucurbita pepo (1/3) ou Serenoa repens (1/3) avec Secale cereale (1/3) et Epilobium angustifolium (1/3) ont un effet additif et synergique sur l'inhibition de la 5 alpha réductase (voir partie pharmacologie). Par ailleurs, la tolérance locale de ces associations est excellente.

Ces associations présentent une meilleure activité pharmacologique que les extraits pris séparément ce qui permet d'en diminuer les doses d'utilisation et limite les risques de toxicité locale ou générale.

La peau est une barrière naturelle qui a pour rôle d'empêcher l'entrée des substances étrangères dans l'organisme. Or, la combinaison d'associations d'extraits de plantes synergiques avec un promoteur d'absorption (Cetiol® HE, Transcutol® ou sphingolipides) permet de transporter les composants actifs précisément au niveau des follicules capillaires, ce qui permet d'améliorer localement les effets pharmacologiques tout en diminuant la toxicité.

Les résultats d'une étude clinique de 4 mois réalisée chez des volontaires sains de sexe masculin ayant un problème de chute de cheveux important (plus de 20% de cheveux en phase télogène) ont montré que l'utilisation sur le cuir chevelu d'une lotion contenant seulement un extrait de Serenoa repens n'a pas d'effet sur les paramètres capillaires. L'application d'une lotion contenant une association d'extraits de plantes synergiques selon l'exemple 1, mais sans promoteur d'absorption, permet de diminuer la proportion de cheveux en phase télogène (-12,2 ± 4,1%) mais n'a pas d'effet sur la densité capillaire ou la vitesse de pousse des cheveux. L'application d'une lotion contenant une association d'extraits de plantes synergiques selon l'exemple 2 contenant un promoteur d'absorption améliore la densité capillaire (+7,2 ± 3,5 cheveux/cm²), augmente légèrement la vitesse de pousse des cheveux et diminue fortement la proportion de cheveux en phase télogène (-20,3 ± 3,3%). L'application sur le cuir chevelu de cette même lotion contenant les extraits de plantes synergiques associée à un complément alimentaire selon l'exemple 6 permet en plus d'améliorer la densité capillaire sans perturber l'effet positif de la lotion sur les autres paramètres.

Ces résultats indiquent clairement que la combinaison de l'association d'extraits de plantes inhibiteurs de la 5 alpha réductase avec un transporteur améliore les effets pharmacologiques de l'association d'extraits de plantes, sans induire d'effet toxique. L'utilisation d'un promoteur d'absorption en juste proportion qui joue le rôle de transporteur permet d'amener les composants actifs précisément au niveau des follicules capillaires, d'inhiber fortement la conversion de la testostérone en dihydrotestostérone au niveau local sans perturber l'équilibre hormonal général.

Aussi, en accord avec les résultats pharmacologiques obtenus, la présente invention concerne donc l'utilisation d'une association contenant au moins trois extraits de plantes inhibiteurs de la conversion de la testostérone en dihydrotestostérone avec ou sans transporteur spécifique (promoteur d'absorption) qui améliore leur biodisponibilité et permet de diminuer leur dose d'utilisation pour la préparation de composition pour lutter contre la chute des cheveux. La composition a l'utilisation revendiquées sont définies aux revendications 1 et 4.

Ces différentes activités nécessitent l'emploi de plusieurs actifs en justes proportions qui doivent êtres apportés localement pour éviter toute toxicité générale. Pour améliorer plus encore son efficacité, une telle composition pour application locale peut également être associée avec une composition par voie orale contenant les éléments indispensables à la pousse des cheveux.

Cette combinaison peut se présenter par exemple sous forme d'une composition pharmaceutique sous forme liquide pour application locale, c'est à dire à titre de médicament, de cure composée d'une lotion capillaire ou d'un shampooing associé ou non à une forme d'apport par voie orale des éléments qui participent à la croissance des cellules folliculaires (acides aminés, vitamines, minéraux).

C'est pourquoi la présente demande a pour objet l'utilisation de compositions contenant une association d'extraits de plantes inhibiteurs de la conversion de la testostérone en dihydrotestostérone, agissant de façon synergique par voie locale pour diminuer la chute des cheveux et comprenant un extrait de Serenoa repens et un extrait d'Urtica dioica et un extrait de Cucurbita pepo en combinaison ou non avec un promoteur d'absorption pour diminuer spécifiquement la chute des cheveux.

On peut formuler ces compositions sous différentes formes galéniques. De telles formes galéniques et leurs modes de préparation sont familières à l'homme de l'art.

Dans des conditions préférentielles de mise en oeuvre de l'invention, on utilise différents principes actifs, notamment des extraits de plantes.

Par "extrait de plante", l'on entend tant des plantes que des parties de plantes, par exemple et de préférence de plante entière et de préférence séchées ou déshydratées, et broyées, ou encore des extraits de telles plantes ou parties de plantes obtenus à l'aide d'au moins un solvant aqueux et/ou organique et se présentant sous une forme liquide classiquement employée en pharmacie ou diététique.

Dans la présente demande et dans ce qui suit, le terme « non solide » appliqué à une composition selon l'invention vise tant les préparations liquides que semi-liquides (visqueuses).

Dans des conditions préférentielles de mise en oeuvre de l'invention, les compositions non solides sont des compositions pharmaceutiques ou cosmétiques.

Selon un mode de réalisation avantageux, la composition non solide est une composition sous forme de lotion et comprend une association d'extraits de plantes capable d'inhiber fortement la conversion de la testostérone en dihydrotestostérone en agissant de façon synergique et au moins un promoteur d'absorption permettant de transporter les agents inhibiteurs précisément au niveau des follicules capillaires.

Selon un mode de réalisation avantageux, cette composition non solide peut être utilisée par voie topique, préférentiellement sur le cuir chevelu.

Cette association peut être utilisée seule ou incorporée dans des lotions, des shampooings, des après-shampooing ou tous autres produits capillaires.

Une composition préférée est caractérisée en ce qu'elle renferme, si l'on se réfère aux extraits de plantes, entre 0,5 et 50% de chaque extrait, de préférence une proportion de 33% dans une base cosmétiquement ou pharmaceutiquement acceptable.

De tels extraits de plante sont par exemple commercialisés par la société BIOSPHERE (France) ou par la société GREENTECH (France).

Les propriétés pharmacologiques de ces produits sont illustrées par les résultats de la partie expérimentale.

En application locale l'association d'extraits de plantes synergiques sous forme de lotion, crème, gel ou émulsion peut être appliquée sur le cuir chevelu au moins une fois par jour, par exemple 1 à 2 ml des compositions présentées en exemple pendant 120 jours.

Selon un mode de réalisation avantageux, la première composition non solide comprend également au moins un promoteur d'absorption, de préférence un Polyol ester d'acides gras (PEG-7 Glyceryl cocoate ou Cetiol® HE, Cognis, France), l'éther monoéthylique du diéthylène glycol purifié (Ethoxydiglycol ou Transcutol®, Gattefossé, France), des sphingolipides tel que des céramides d'origine végétale ou animale.

Une composition ci-dessus préférée est caractérisée en ce qu'elle renferme, si l'on se réfère aux promoteurs d'absorption, entre 0,05 et 20%, dans une base cosmétiquement ou pharmaceutiquement acceptable.

Ces compositions notamment pharmaceutiques ou cosmétiques peuvent être, par exemple, liquides ou semi-liquides et se présenter sous les formes pharmaceutiques couramment utilisées en médecine humaine ; elles sont préparées selon les méthodes usuelles. Le ou les principes actifs peuvent y être incorporés à des excipients habituellement employés dans les compositions pharmaceutiques, tels que les véhicules aqueux ou non, les divers agents mouillants, les conservateurs, les épaississants.

Le procédé de préparation des compositions ci-dessus décrites est caractérisé en ce que l'on mélange, selon des méthodes connues en elles-mêmes, le ou les principes actifs avec des excipients ou solvants acceptables, notamment pharmaceutiquement ou cosmétiquement acceptables.

Dans des conditions préférentielles de mise en oeuvre de l'invention, la composition solide utilisée par voie orale est une composition sous forme de poudre pour apporter les nutriments indispensables à la croissance du cheveu.

Dans des conditions préférentielles de mise en oeuvre de l'invention, la composition solide est une composition pour une utilisation par voie orale.

Selon un mode de réalisation avantageux, cette composition solide résulte de l'utilisation, seuls ou en association, d'agents permettant un apport en acides aminés, et notamment en acides aminés soufrés, d'agents permettant un apport en vitamines et en minéraux.

Dans des conditions préférentielles de mise en oeuvre l'agent permettant un apport en acides aminés pourra être choisi parmi le groupe des protéines.

Ainsi, on peut trouver dans les compositions solides selon l'invention, une ou plusieurs substances capables d'apporter des acides aminés, et notamment, les protéines végétales ou animales, de préférence les hydrolysats de céréales, les hydrolysats de blé, de soja, de préférence les protéines de lait, de préférence les levures.

Dans des conditions préférentielles de mise en oeuvre l'agent permettant un apport en acides aminés pourra être choisi parmi le groupe des acides aminés purifiés, de préférence la L-cystéine, la L-cystine, la L-méthionine.

Dans des conditions préférentielles de mise en oeuvre les agents permettant un apport en minéraux pourront être choisis parmi les formes d'apport usuelles synthétiques ou naturelles de minéraux, de préférence les formes d'apport en zinc, notamment l'acétate de zinc, une source ou un extrait naturel riche en zinc, de préférence les formes d'apport en magnésium, notamment l'oxyde de magnésium, une source ou un extrait naturel riche en sélénium, de préférence les formes d'apport en sélénium, notamment les levures enrichies en sélénium, de préférence les formes d'apport en cuivre, notamment l'oxyde de cuivre, une source ou un extrait naturel riche en cuivre, de préférence les formes d'apport en fer, notamment le sulfate de fer, une source ou un extrait naturel riche en fer.

Dans des conditions préférentielles de mise en oeuvre les agents permettant un apport en vitamines pourront être choisis parmi les formes d'apport usuelles synthétiques ou naturelles de vitamines, de préférence les formes d'apport en vitamine B5, notamment le pantothénate de calcium, une source ou un extrait naturel riche en vitamine B5, de préférence les formes d'apport en vitamine B8 ou biotine, une source ou un extrait naturel riche en vitamine B8, de préférence les formes d'apport en vitamine A, notamment le bêta carotène, une source ou un extrait naturel riche en vitamine A.

De tels agents sont par exemple commercialisés par la société QUIMDIS (France) ou par la société COOPER (Coopération Pharmaceutique Française, France).

La dose usuelle, variable selon la composition choisie peut être par exemple de 200 milligrammes à 6 grammes par jour par voie orale chez l'homme ou représenter entre 50 et 200% des Apports Journaliers Recommandés (AJR) pour les minéraux et les vitamines, pendant 120 à 240 jours, plus particulièrement une quantité journalière de 300 à 350 milligrammes pendant une période de 120 jours.

Les exemples qui suivent illustrent la présente demande. Ces exemples de compositions pour inhiber la conversion de la testostérone en dihydrotestostérone au niveau du cuir chevelu et apporter aux cheveux les éléments indispensables à leur croissance ont été développés sur la base des résultats obtenus au cours des études pharmacologiques. Certaines compositions ont ensuite été soumises à des expérimentations cliniques.

### EXEMPLE 1

On a préparé des flacons de lotion de différentes contenances répondant à la formule :

| | |
|---|---|
| Extrait de Serenoa repens | 22,5% |
| Extrait d'Urtica dioica | 22,5% |
| Extrait de pépins de Cucurbita pepo | 22,5% |
| Excipients | qsp 100% |

Posologie : pendant 120 jours, appliquer 2 à 3 ml de lotion chaque soir et masser le cuir chevelu pour répartir le produit. Laisser agir la lotion au cours de la nuit.

### EXEMPLE 2

On a préparé des flacons de composition non solide de différentes contenances répondant à la formule :

| | |
|---|---|
| Extrait de Serenoa repens | 22,5% |
| Extrait d'Urtica dioica | 22,5% |
| Extrait de pépins de Cucurbita pepo | 22,5% |
| Cetiol® HE | 5,0% |
| Excipients | qsp 100% |

Posologie : 1 ou 2 applications de 2 à 3 ml du produit au moins une fois par semaine pendant 16 semaines.

### EXEMPLE 3

On a préparé des flacons de composition non solide de différentes contenances répondant à la formule :

| | |
|---|---|
| Extrait de Serenoa repens | 22,5% |
| Extrait d'Urtica dioica | 22,5% |
| Extrait de pépins de Cucurbita pepo | 22,5% |
| Cetiol® HE | 4,5% |
| Sphingolipides de blé | 0,5% |
| Excipients | qsp 100% |

Posologie : 1 ou 2 applications de 2 à 3 ml du produit au moins une fois par semaine pendant 16 semaines.

### EXEMPLE 4

On a préparé des flacons de composition non solide de différentes contenances répondant à la formule :

| | |
|---|---|
| Extrait de Serenoa repens | 10,0% |
| Extrait d'Urtica dioica | 10,0% |
| Extrait de pépins de Cucurbita pepo | 10,0% |
| Extrait d'Epilobium angustifolium | 10,0% |
| Transcutol® CG | 2,0% |
| Excipients | qsp 100% |

Posologie : 1 ou 2 applications de 0,5 à 1 ml du produit au moins une fois par semaine pendant 16 semaines.

On a préparé des gélules de complément alimentaire de 374 milligrammes répondant à la formule :

| | milligrammes / gélule |
|---|---|
| Protéines de blé | 242,00 mg |
| Lactose | 100,66 mg |
| Acétate de zinc | 25,19 mg |
| Pantothénate de calcium | 3,00 mg |
| Biotine | 0,15 mg |
| Excipients | qsp 374,00 mg |

Posologie: 1 gélule avec un verre d'eau, le matin de préférence.

### EXEMPLE 5

On a préparé des flacons de composition non solide de différentes contenances répondant à la formule :

| | |
|---|---|
| Extrait de Serenoa repens | 10,00% |
| Extrait d'Urtica dioica | 10,00% |
| Extrait de pépins de Cucurbita pepo | 10,00% |
| Extrait de Secale cereale | 10,00% |
| Transcutol® CG | 2,50% |
| Pantothénate de calcium | 1,00% |
| Biotine | 0,50% |
| Protéines de blé | 0,50% |
| Sphingolipides | 0,50% |
| Excipients | qsp 100% |

Posologie : 1 ou 2 applications de 2 à 3 ml du produit au moins une fois par semaine pendant 16 semaines.

On a préparé des gélules de complément alimentaire de 330 milligrammes répondant à la formule :

| | milligrammes / gélule |
|---|---|
| Levure de bière | 100,00 mg |
| Huile de poisson encapsulée | 50,00 mg |
| Acétate de zinc | 26,00 mg |
| Levure de bière enrichie en sélénium | 18,75 mg |
| Acide ascorbique | 15,00 mg |
| Acétate de tocophérol | 5,00 mg |
| Pantothénate de calcium | 3,00 mg |
| Bêta carotène | 2,40 mg |
| Biotine | 0.07 mg |
| Excipients | qsp 330 mg |

Posologie: 2 gélules avec un verre d'eau, le matin de préférence.

### ETUDE PHARMACOLOGIQUE

### Recherche d'associations d'agents capables d'inhiber la conversion de la testostérone en dihydrotestostérone.

Les 5 alpha réductases de type I (5aRI) et de type II (5aRII) catalysent la conversion de la testostérone en dihydrotestostérone.

Une étude biochimique des cinétiques de conversion a été réalisée en présence ou en l'absence de différents extraits de plantes, seuls ou en mélange, selon une méthode adaptée de Mc Nultry et al. (J. Steroid Biochem Mol Biol, 2000, 72, 1-2, 13-21). En comparant l'activité ainsi obtenue à l'activité de base de l'enzyme, il a été possible d'établir des indices d'activité et d'évaluer l'activité inhibitrice des différents extraits.

Les résultats suivants ont été obtenus (moyenne de 3 expériences, n = 3 par expérience) :

| Extrait ou association testée | Inhibition de la 5 alpha réductase | |
|---|---|---|
| | 5aRI | 5aRII |
| - Serenoa repens seul (100%) | 48% | 49% |
| - Urtica dioica seul (100%) | 36% | 29% |
| - Cucurbita pepo seul (100%) | 32% | 28% |
| - E. angustifolium seul (100%) | 34% | 33% |
| - Secale cereale seul (100%) | 28% | 28% |
| - Allium sativum seul (100%) | 18% | 10% |
| - G. robertianum seul (100%) | 15% | 7% |
| - Serenoa repens + Urtica dioica (50/50) | 74% | 72% |
| - Serenoa repens + Cucurbita pepo (50/50) | 68% | 68% |
| - Serenoa repens + E. angustifolium (50/50) | 72% | 78% |
| - Serenoa repens + Secale cereale (50/50) | 63% | 65% |
| - Serenoa repens + Allium sativum (50/50) | 34% | 24% |
| - Serenoa repens + G. robertianum (50/50) | 28% | 22% |
| - Serenoa repens + Urtica dioica + Cucurbita pepo (1/3 : 1/3 : 1/3) | 88% | 86% |
| - Serenoa repens + Secale cereale + E. angustifolium (1/3 : 1/3 : 1/3) | 83% | 85% |

| | | |
|---|---|---|
| E. angustifolium = Epilobium angustifolium | | |
| G. robertianum = Geranium robertianum | | |

Les résultats de cette étude montrent que les agents testés inhibent la 5 alpha réductase de type I et de type II de façon variable. L'association de l'extrait de Serenoa repens avec l'extrait d'Allium sativum ou de Géranium robertianum ne permet pas d'augmenter l'inhibition de la 5 alpha réductase. Par contre, l'association de l'extrait de Serenoa repens avec l'extrait d'Urtica dioica, de Cucurbita pepo , de Secale cereale ou d'Epilobium angustifolium permet une plus forte inhibition que l'extrait de Serenoa repens seul. L'inhibition de la 5 alpha réductase par l'association de l'extrait de Serenoa repens, d'Urtica dioica et de Cucurbita pepo ou par l'association de Serenoa repens, de Secale cereale et d'Epilobium angustifolium est plus forte que celle des extraits pris séparément ou que des associations de seulement deux extraits de plantes.

### Recherche d'associations d'agents capables d'inhiber la conversion de la testostérone en dihydrotestostérone à l'aide de techniques in vitro.

L'inhibition de la 5 alpha réductase a été mesurée à l'aide d'une suspension cellulaire contenant des noyaux, des mitochondries et des microsomes de prostate humaine ou du rat selon une méthode adaptée de Hartmann et al. (Eur. J. Med Chem., 1994, 29, 807-817). L'inhibition a été mesurée en incubant l'enzyme et de la [1,2-³H]-testostérone en présence d'extraits de plantes. La solution résultante a été séparée par HPLC et les aires correspondantes aux pics de la testostérone ou de son métabolite, la dihydrotestostérone, ont été mesurées. Le rapport entre l'aire des pics a fourni une mesure quantitative de l'inhibition.

L'extrait de Serenoa repens seul présentait une concentration capable d'inhiber l'activité de l'enzyme à 50% ou IC50 de 0,44 µM, l'extrait d'Urtica dioica seul une IC50 de 1,27 µM et l'extrait de Cucurbita pepo seul une IC50 de 1,68 µM. Lorsqu'on utilise une association des trois extraits, l'IC 50 du mélange est de 0,25 µM, ce qui montre que l'association présente une meilleure activité que les trois extraits pris séparément.

Comme il n'existe pas de modèle animal fiable pour évaluer l'activité des produits sur la chute des cheveux, certaines compositions présentées en exemple ont directement fait l'objet de tests de tolérance et d'un test clinique.

### Etudes de tolérance :

Les études de tolérance locale cutanée et de tolérance oculaire chez le lapin réalisées selon la méthode officielle pour l'appréciation de l'agressivité superficielle cutanée (Arrêté du 11 mai 1993) et la méthode officielle pour l'évaluation de l'irritation oculaire (Arrêté du 9 juin 1992) sous couvert d'assurance qualité montrent que les préparations non solides présentées en exemple sont non irritantes.

Les études de toxicité aiguë chez le rat réalisées selon les lignes directrices de l'OCDE n° 401 sous couvert d'assurance qualité montrent que les préparations solides présentées en exemple sont non toxiques à la dose de 5 g/kg.

### ETUDE CLINIQUE

### Comparaison de l'activité de quatre traitements sur les paramètres capillaires, la tolérance locale et systémique chez le volontaire sain.

Cette étude clinique a été réalisée en France par un laboratoire accrédité par le Ministère de la Recherche. Soixante volontaires entre 18 et 60 ans et ayant au moins 20% de cheveux en phase télogène ont été répartis en 4 groupes. Les volontaires ne devaient pas utiliser de traitement anti-chute pendant, ni les 3 mois précédant l'étude.

| Groupe | Traitement |
|---|---|
| 1 | Lotion contenant 67,5% d'un extrait de Serenoa repens. |
| 2 | Lotion contenant l'association synergique de Serenoa repens (1/3) Urtica dioica (1/3) et Cucurbita pepo (1/3) selon l'exemple 1. |
| 3 | Lotion contenant l'association synergique de Serenoa repens (1/3) Urtica dioica (1/3) et Cucurbita pepo (1/3)avec un promoteur d'absorption (Cetiol HE 5%) selon l'exemple 2. |
| 4 | Lotion contenant l'association synergique de Serenoa repens (1/3) Urtica dioica (1/3) et Cucurbita pepo (1/3) selon l'exemple 2 en combinaison avec le complément alimentaire selon l'exemple 4. |

En fonction du groupe d'étude, il a été demandé aux volontaires d'appliquer 1,5 à 2 ml de lotion en friction chaque soir avant le coucher (groupes 1, 2, 3 et 4), sans rincer, le cas échéant de prendre 1 gélule de la composition solide le matin avec un verre d'eau (groupe 4), pendant 120 jours.

Des vidéotrichogrammes réalisés au jour 0-2 et au jour 120-122 ont permis de déterminer le nombre de cheveux sur chaque zone étudiée, la proportion de cheveux en phase télogène et la vitesse de pousse des cheveux en phase anagène pour chaque volontaire. Les mesures ont été réalisées à des endroits identiques aux jours 0-2 et aux jour 120-122.

Après 122 jours d'utilisation, les résultats suivant ont été obtenus :

| | Densité capillaire par rapport au jour 0 (cheveux / cm²) | Vitesse de pousse des cheveux par rapport au jour 0 (mm/j) | Proportion de cheveux en phase télogène par rapport au jour 0 (%) |
|---|---|---|---|
| Groupe 1 | Pas d'effet | Pas d'effet | -3,2 ± 2,1 % |
| Groupe 2 | Pas d'effet | Pas d'effet | -12,2 ± 4,1% |
| Groupe 3 | +7,2 ± 3,5 | Légère augmentation | 20,3 ± 3,3% 20,3 ± 3,3% |
| | | | (p<0,07) |
| Groupe 4 | +9 ± 5(p=0,07) | Légère augmentation | 21,5 ± 1,4% 21,5 ± 1,4% |
| | | | (p<0,001) |

Si on compare les quatre traitements entre eux, on observe que la lotion contenant un seul extrait a une faible activité sur le pourcentage de cheveux en phase télogène et n'a pas d'effet sur la densité capillaire et la vitesse de pousse des cheveux. L'utilisation de la lotion contenant une association de trois extraits de plantes synergiques sans promoteur d'absorption permet de diminuer le pourcentage de cheveux en phase télogène de -12,2 ± 4,1%. Par contre, l'utilisation de la lotion seule contenant l'association de trois extraits de plantes synergiques avec un promoteur d'absorption permet de diminuer le pourcentage de cheveux en phase télogène de -20,3 ± 3,3% (p<0,007) et en plus, d'augmenter légèrement la densité capillaire et la vitesse de pousse des cheveux. Ces résultats indiquent que les lotions contenant l'association de trois extraits de plantes synergiques contiennent des substances actives qui agissent *in vivo*, probablement en inhibant la 5 alpha réductase au niveau du cuir chevelu et qu'il existe une réelle synergie d'action entre les trois extraits utilisés. Ces résultats montrent également que l'utilisation d'un promoteur d'absorption permet d'augmenter l'efficacité des composés inhibiteurs de la 5 alpha réductase. L'application locale de la lotion contenant l'association de trois extraits de plantes synergiques avec un promoteur d'absorption en combinaison avec un complément alimentaire contenant des éléments indispensables à la croissance des cellules folliculaires ne modifie pas l'effet de la lotion seule sur la vitesse de pousse et la proportion de cheveux en phase télogène mais permet d'améliorer la densité capillaire.

Ces résultats montrent qu'il existe une synergie d'action entre les extraits de plantes inhibiteurs de la 5 alpha réductase présents dans les lotions utilisées, que leur activité pharmacologique peut être fortement améliorée en utilisant un transporteur ou un promoteur d'absorption ainsi qu'avec un complément alimentaire apportant les éléments indispensables au métabolisme des cellules des follicules capillaires.

En raison de sa forte capacité de pénétration, la lotion contenant les associations d'extraits de plantes synergiques en combinaison avec des transporteurs a agi spécifiquement sur les follicules capillaires et a permis de diminuer la chute des cheveux en retardant l'entrée des follicules en phase télogène (allongement de la phase anagène).

Aucun des participants ayant utilisé les compositions n'a fait état de démangeaisons ou d'irritation locale. L'absence d'effets secondaires systémiques indique la stabilité du taux sanguin de testostérone et l'absence de perturbation de l'équilibre hormonal général.

Une diminution du pourcentage de cheveux en phase télogène traduit une diminution de la chute des cheveux. Une diminution de 25% du nombre de cheveux en phase télogène est considérée comme un maximum pour permettre le rétablissement d'une répartition normale des cheveux au sein des différentes phases. L'utilisation d'une lotion contenant une association d'extraits de plantes synergiques en combinaison avec un promoteur d'absorption procure d'excellents résultats car elle permet une diminution de la proportion de cheveux en phase télogène de -20,3 ± 3,3%.

Plus de 80% des participants ont obtenu une diminution de la chute des cheveux en seulement 120 jours, contre 20 à 30% seulement avec les produits classiques habituellement employés sur une période de 6 à 12 mois.

Ainsi, la chute des cheveux peut être diminuée efficacement en un temps très court et sans effets secondaires en utilisant une association d'extraits de plantes synergiques qui ont pour effet d'inhiber la conversion de la testostérone en dihydrotestostérone au niveau du cuir chevelu en combinaison avec un promoteur d'absorption.

## Revendications

1. Composition destinée à diminuer la chute des cheveux **caractérisée en ce qu'**elle comprend un extrait de Serenoa repens, un extrait d'Urtica dioica et un extrait de Cucurbita pepo.

2. Composition selon la revendication 1 **caractérisée en ce qu'**elle comprend un promoteur d'absorption cutanée choisi dans le groupe constitué par les polyols esters d'acides gras, les éthers monoéthyliques du diéthylène glycol, les sphingolipides.

3. Composition selon l'une quelconque des revendications 1 ou 2 **caractérisée ne ce qu**'elle comprend un excipient pharmaceutiquement et/ou cosmétiquement acceptable.

4. Utilisation non thérapeutique d'une composition selon l'une quelconque des revendications précédentes en application locale pour diminuer la chute des cheveux.

## Patentansprüche

1. Zusammensetzung mit dem Ziel der Verminderung von Haarausfall, die als besondere Kennzeichen einen Extrakt von Serenoa repens, einen Extrakt von Urtica dioica und einen Extrakt von Cucurbita pepo enthält.

2. Zusammensetzung gemäß Patentanspruch 1, **gekennzeichnet durch** einen Promotor für die Hautabsorption aus der Gruppe der Polyolester der Fettsäuren, der Monoethylenether des Diethylenglycols sowie der Sphingolipide.

3. Zusammensetzung gemäß Patentanspruch 1 oder 2, **gekennzeichnet durch** eine pharmakologisch und/oder kosmetisch akzeptablen Grundmasse.

4. Nichttherapeutische Verwendung einer Zusammensetzung gemäß einem der vorherigen Patentansprüche zur örtlichen Anwendung zum Vermindern von Haarausfall.

## Claims

1. Composition for reducing hair loss, **characterised in that** it comprises a Serenoa repens extract, an Urtica dioica extract and a Cucurbita pepo extract.

2. Composition according to claim 1, **characterised in that** it comprises a cutaneous absorption promoter selected from the group consisting of fatty acid ester polyols, diethylene glycol monoethyl ethers and sphingolipids.

3. Composition according to either claim 1 or claim 2, **characterised in that** it comprises a pharmaceutically and/or cosmetically acceptable excipient.

4. Non-therapeutic use of a composition according to any one of the preceding claims applied locally for reducing hair loss.
